(19) Europäisches Patentamt
European Patent Office
Office européen des brevets

(11) **EP 3 000 397 A1**

(12) **DEMANDE DE BREVET EUROPEEN**

(43) Date de publication:
**30.03.2016 Bulletin 2016/13**

(51) Int Cl.:
*A61B 5/117* (2006.01)     *G06K 9/00* (2006.01)
*A61B 5/00* (2006.01)      *H04N 7/18* (2006.01)
*G06K 9/20* (2006.01)

(21) Numéro de dépôt: **15182665.8**

(22) Date de dépôt: **27.08.2015**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**MA**

(30) Priorité: **26.09.2014 FR 1459133**

(71) Demandeur: **Morpho
92130 Issy-les-Moulineaux (FR)**

(72) Inventeurs:
• **ROUH, Alain
92130 Issy-les-Moulineaux (FR)**
• **MALRAT, Benoît
92130 Issy-les-Moulineaux (FR)**

(74) Mandataire: **Laïk, Eric et al
Cabinet Beau de Loménie
158, rue de l'Université
75340 Paris Cedex 07 (FR)**

(54) **DISPOSITIF DESTINÉ À ÊTRE UTILISÉ POUR IDENTIFIER OU AUTHENTIFIER UN SUJET**

(57) L'invention concerne un dispositif (1) destiné à être utilisé pour identifier ou authentifier un sujet (S) positionné dans un volume d'acquisition (V) à partir d'au moins une caractéristique biométrique du sujet (S), le dispositif (1) comportant successivement, de manière décalée le long d'un axe (X) de placement de caméras, une première caméra (21), une deuxième caméra (22) et une troisième caméra (23), les axes optiques ($A_{21}$ ; $A_{22}$) des première et deuxième caméras formant entre eux un angle strictement inférieur à 10° et l'axe optique ($A_{23}$) de la troisième caméra intersectant les axes optiques ($\hat{A}_{21}$ ; $A_{22}$) des première et deuxième caméras, les axes optiques ($A_{21}$ ; $A_{22}$) des première et deuxième caméras formant chacun un angle inférieur ou égal à 5° avec un axe normal (Y) perpendiculaire à l'axe (X) de placement des caméras.

FIG.1

**Description**

Arrière-plan de l'invention

L'invention concerne des dispositifs utiles pour l'identification ou l'authentification d'un sujet ainsi que des procédés mettant en oeuvre de tels dispositifs.

On connaît des dispositifs de reconnaissance faciale comportant plusieurs caméras afin de couvrir le volume d'acquisition nécessaire à l'adaptation morphologique et au confort ergonomique, les caméras étant disposées de façons à couvrir le champ le plus large possible et avec un recouvrement des champs des caméras suffisant. Dans de tels dispositifs, les axes optiques des caméras peuvent être parallèles et horizontaux comme dans le système SmartGate®. Ces systèmes permettent par exemple de réaliser l'authentification d'un sujet en confirmant ou infirmant la prétendue identité d'un sujet à partir d'une ou plusieurs images de ce dernier prises par les caméras et après comparaison avec des données de référence stockées dans un support de stockage d'information.

D'autres systèmes peuvent mettre en oeuvre des caméras dont les axes optiques sont concourants, afin de maximiser le volume dans lequel le sujet est dans le champ des différentes caméras. Ces derniers systèmes permettent de réaliser une triangulation ainsi qu'une reconstruction 3D de la position d'une zone du visage d'un sujet du fait de l'utilisation de caméras ayant des champs de vue qui se recouvrent.

Toutefois, les deux types de systèmes décrits plus hauts peuvent ne pas être optimaux pour constituer un système multi-biométrique de reconnaissance faciale 2D et de reconnaissance d'iris utilisant une localisation 3D des yeux par triangulation.

En outre, les systèmes dont les axes optiques sont concourants comportent au moins une caméra dont l'axe optique est dirigé vers le haut. Cela peut conduire à un éblouissement de cette caméra par des éclairages situés en hauteur ou par le soleil et donc à une diminution des performances d'identification.

Par ailleurs, il peut être souhaitable de réduire au maximum l'encombrement des dispositifs d'identification ou d'authentification d'un sujet notamment lorsque ces dispositifs sont destinés à être utilisés dans des zones aéroportuaires.

Il existe donc un besoin pour obtenir des dispositifs utiles pour l'identification ou l'authentification d'un sujet pouvant être utilisés pour réaliser de manière satisfaisante à la fois une reconnaissance faciale du sujet ainsi qu'une détermination de la position 3D d'une zone du visage du sujet.

Il existe aussi un besoin pour obtenir des dispositifs utiles pour l'identification ou l'authentification d'un sujet dont les performances sont peu affectées par les conditions lumineuses ambiantes.

Il existe aussi un besoin pour obtenir des dispositifs utiles pour l'identification ou l'authentification d'un sujet présentant un encombrement limité.

Il existe encore un besoin pour obtenir des dispositifs utiles pour l'identification ou l'authentification d'un sujet présentant un temps de calcul et un coût de fonctionnement réduits.

Objet et résumé de l'invention

A cet effet, l'invention propose, selon un premier aspect, un dispositif destiné à être utilisé pour identifier ou authentifier un sujet positionné dans un volume d'acquisition à partir d'au moins une caractéristique biométrique du sujet, le dispositif comportant successivement, de manière décalée le long d'un axe de placement de caméras, une première caméra, une deuxième caméra et une troisième caméra, les axes optiques des première et deuxième caméras formant entre eux un angle strictement inférieur à 10° et l'axe optique de la troisième caméra intersectant les axes optiques des première et deuxième caméras.

Dans un procédé d'identification d'un sujet, le dispositif peut recevoir des images d'un sujet inconnu et, après comparaison avec des données de référence stockées dans un support de stockage d'information, déterminer l'identité dudit sujet. Dans un procédé d'authentification d'un sujet, le dispositif confirme ou infirme la prétendue identité d'un sujet à partir d'une ou plusieurs images de ce dernier et après comparaison avec des données de référence stockées dans un support de stockage d'information. L'identification ou l'authentification peuvent être réalisées de manière automatique. Le support de stockage d'informations stockant les données de référence peut être intégré au dispositif. En variante, le support de stockage d'informations peut être présent dans un système informatique externe au dispositif, le dispositif étant alors configuré pour échanger des données avec ledit système informatique.

Une caractéristique biométrique d'un sujet est une caractéristique biologique de ce sujet utile pour l'identifier. On peut par exemple citer comme caractéristiques biométriques les caractéristiques relatives aux empreintes digitales du sujet, à l'iris du sujet ou encore la forme du visage du sujet.

Par « l'axe optique de la troisième caméra intersectant les axes optiques des première et deuxième caméras », il faut comprendre que l'axe optique de la troisième caméra n'est pas parallèle aux axes optiques des première et deuxième caméras, l'axe optique de la troisième caméra étant dirigé vers les axes optiques des première et deuxième caméras afin d'être sécant avec ces derniers.

**EP 3 000 397 A1**

Le dispositif selon l'invention présente deux fonctions. D'une part, il peut être utilisé pour réaliser une reconnaissance faciale 2D à l'aide de tout ou partie des images issues des première, deuxième et troisième caméras. D'autre part, il permet de déterminer la position d'une zone du visage du sujet par triangulation à l'aide d'au moins deux des première, deuxième et troisième caméras. En particulier, il est possible de déterminer la position d'une zone du visage du sujet par triangulation à l'aide de la troisième caméra et de l'une au moins des première et deuxième caméras. En variante, il est possible de déterminer la position d'une zone du visage du sujet par triangulation uniquement à l'aide de la première caméra et de la deuxième caméra.

Ainsi, le dispositif selon l'invention comporte une pluralité de caméras ayant une disposition particulière utiles pour réaliser de manière satisfaisante à la fois une reconnaissance faciale à partir d'images 2D d'un sujet ainsi qu'une détermination de la position d'au moins une zone du visage du sujet, par exemple des yeux, par stéréoscopie. Comme détaillé plus bas, un pilotage d'un système de prise de vue d'iris ou une reconnaissance faciale 3D pourra être réalisé à partir de la position 3D d'une zone du visage du sujet déterminée par triangulation.

Dans un exemple de réalisation, les axes optiques des première et deuxième caméras peuvent former entre eux un angle inférieur ou égal à 5°, voire être sensiblement parallèles entre eux, voire être parallèles entre eux (angle de 0°).

De préférence, les axes optiques des première et deuxième caméras peuvent chacun former un angle inférieur ou égal à 10°, par exemple inférieur ou égal à 5°, avec un axe normal perpendiculaire à l'axe de placement des caméras. Dans un exemple de réalisation, les axes optiques des première et deuxième caméras peuvent chacun être parallèles à l'axe normal.

De telles configurations sont avantageuses car elles permettent de minimiser l'angle formé entre la normale au visage du sujet et les axes optiques des première et deuxième caméras afin d'obtenir de très bonnes performances en reconnaissance faciale 2D.

Dans un exemple de réalisation, les axes optiques des première, deuxième et troisième caméras peuvent ne pas être concourants (i.e. ne pas se couper au même point).

Dans un exemple de réalisation, l'axe de placement des caméras peut être sensiblement vertical. Une telle configuration permet avantageusement d'améliorer encore les performances et l'ergonomie du dispositif. Ainsi, le dispositif peut être tel que :

- la première caméra soit placée à une première hauteur,
- la deuxième caméra soit placée à une deuxième hauteur supérieure à la première hauteur, et
- la troisième caméra soit placée à une troisième hauteur supérieure à la deuxième hauteur.

Sauf mention contraire, les hauteurs des éléments du dispositif (caméras, système de prise de vue d'iris...) sont mesurées par rapport et perpendiculairement à la surface de positionnement du sujet dans le volume d'acquisition. La surface de positionnement correspond à la surface sur laquelle le sujet est destiné à être présent lors de l'identification ou de l'authentification. Ainsi, la hauteur de cette surface est prise comme hauteur de référence (égale à 0).

Dans un exemple de réalisation, l'axe optique de la troisième caméra et l'un au moins des axes optiques des première et deuxième caméras s'étendent en se rapprochant d'une surface de positionnement sur laquelle le sujet est destiné à être présent pour procéder à l'identification ou à l'authentification. En particulier dans ce cas, l'un des axes optiques des première et deuxième caméras peut être parallèle à l'axe normal. En variante, chacun des axes optiques des première et deuxième caméras peut s'étendre en se rapprochant d'une surface de positionnement sur laquelle le sujet est destiné à être présent pour procéder à l'identification ou à l'authentification.

En variante, l'axe optique de la troisième caméra peut s'étendre en se rapprochant d'une surface de positionnement sur laquelle le sujet est destiné à être présent pour procéder à l'identification ou à l'authentification et les axes optiques des première et deuxième caméras peuvent chacun être parallèles à l'axe normal.

Des configurations dans lesquelles les axes optiques des caméras sont soit parallèles à l'axe normal (i.e. horizontaux dans le cas d'un axe de placement des caméras s'étendant en hauteur), soit s'étendent en se rapprochant de la surface de positionnement (i.e. dirigés vers les hauteurs décroissantes selon l'axe de placement des caméras) sont telles qu'aucun des axes optiques des caméras n'est dirigé vers le haut (i.e. vers les hauteurs croissantes selon l'axe de placement des caméras).

Ces configurations permettent avantageusement de conférer au dispositif une très bonne robustesse vis-à-vis des conditions lumineuses du fait de l'absence de caméra dont l'axe optique est orienté vers le haut qui serait susceptible d'être éblouie par des sources de lumières artificielles situées en hauteur ou par le soleil.

De préférence, la superposition des champs des première et deuxième caméras peut recouvrir l'intégralité du volume d'acquisition et les conditions suivantes peuvent être vérifiées :

- l'angle $\alpha_{23}$ formé entre l'axe optique de la troisième caméra et un axe normal perpendiculaire à l'axe de placement des caméras est tel que :

3

$$(\text{arctg}(h'_{23}/d) - \beta_{23}/2) \leq \alpha_{23} \leq 1{,}1*(\text{arctg}(h'_{23}/d) - \beta_{23}/2), \text{ et}$$

- l'angle $\beta_{23}$ de champ selon la hauteur de la troisième caméra est tel qu'il vérifie la condition suivante :

$$\beta_{23} \geq 2 * (\alpha_{23} + \text{arctg}((h_v - h'_{23})/d)),$$

où $h'_{23}$ désigne la hauteur de la troisième caméra mesurée par rapport à la hauteur de la frontière inférieure du volume d'acquisition, $h_v$ désigne la hauteur du volume d'acquisition, d désigne la distance mesurée selon l'axe normal entre la troisième caméra et le volume d'acquisition.

Par « superposition des champs des première et deuxième caméras », on entend l'union (i.e. l'addition) de ces champs. La condition mathématique ci-dessus imposée à $\beta_{23}$ lorsqu'elle est combinée à la condition mathématique ci-dessus imposée à $\alpha_{23}$ implique notamment que le champ de la troisième caméra soit suffisant pour recouvrir à la fois les parties inférieure et supérieure du volume d'acquisition.

De telles valeurs pour l'inclinaison de l'axe optique de la troisième caméra correspondent à une situation optimale dans laquelle cette inclinaison est suffisamment grande pour permettre une triangulation avec l'une au moins des première et deuxième caméras dans l'intégralité du volume d'acquisition, cette inclinaison étant, en outre, suffisamment faible pour éviter de faire un angle trop élevé avec la direction de la normale au visage des sujets les plus grands. En outre, l'axe optique d'une troisième caméra qui présente de telles valeurs d'inclinaison forme avantageusement un angle réduit avec la normale au visage de sujets de petites tailles dont le visage serait orienté vers le haut dans le cas de la lecture d'un écran situé sur le dispositif par exemple. De telles valeurs d'inclinaison pour l'axe optique de la troisième caméra permettent aussi de limiter le risque d'éblouissement de cette caméra par des sources de lumières situées en hauteur comme des éclairages plafonniers ou par le soleil.

Ainsi, de telles valeurs pour l'inclinaison de l'axe optique de la troisième caméra permettent, d'une part, d'obtenir des performances optimales en termes de triangulation à l'aide de la troisième caméra du fait de la couverture de l'intégralité du volume d'acquisition par le champ de la troisième caméra. D'autre part, une telle inclinaison de l'axe optique de la troisième caméra reste relativement limitée ce qui est avantageux dans le cas où la troisième caméra assure, en plus de la fonction de caméra stéréoscopique, la fonction de caméra 2D pour la reconnaissance faciale.

De préférence, $\alpha_{23}$ peut être égal à $(\text{arctg}(h'_{23}/d) - \beta_{23}/2)$.

Dans un exemple de réalisation, le dispositif peut comporter, en plus des première, deuxième et troisième caméras, un système de prise de vue d'iris intégré. Ce système est un système de prise de vue de la texture d'au moins un iris et est par exemple en communication avec un système de reconnaissance d'iris. Ledit système de reconnaissance d'iris est configuré pour réaliser la reconnaissance d'au moins un iris des yeux du sujet à partir d'au moins une image d'un ou des iris des yeux du sujet prise par le système de prise de vue d'iris. Ainsi, le système de prise de vue d'iris peut être configuré pour transmettre des données à un système de reconnaissance d'iris.

Le système de reconnaissance d'iris peut être intégré au dispositif. En variante, le système de reconnaissance d'iris est situé à l'extérieur du dispositif. Le système de reconnaissance d'iris comporte un système d'analyse d'images. Ce système d'analyse d'images peut permettre, à partir d'au moins une image d'un ou des iris des yeux du sujet prise par le système de prise de vue d'iris, d'obtenir des données associées à la texture d'un ou des iris des yeux du sujet, ces données étant utiles pour réaliser la reconnaissance d'au moins un iris des yeux du sujet.

Dans un exemple de réalisation, les trois conditions suivantes peuvent être vérifiées :

- la première hauteur est comprise entre 130 cm et 150 cm,
- la deuxième hauteur est comprise entre 150 cm et 170 cm, et
- la troisième hauteur est comprise entre 180 cm et 200 cm.

La présente invention vise également une porte automatique équipée d'un dispositif tel que décrit plus haut. La présente invention vise également un sas de passage équipé d'un dispositif tel que décrit plus haut.

La présente invention vise également un procédé d'identification ou d'authentification d'un sujet mettant en oeuvre un dispositif, une porte automatique ou un sas de passage tels que décrits plus haut, le procédé comportant les étapes suivantes :

a) positionnement du sujet dans le volume d'acquisition, et
b) réalisation d'une reconnaissance faciale 2D du sujet à partir d'une ou plusieurs images du sujet prises par l'une au moins des première, deuxième et troisième caméras.

Cette reconnaissance faciale 2D peut être effectuée par le dispositif ou, en variante, par un système informatique externe au dispositif à partir de données associées à au moins une image du sujet prise par l'une au moins des caméras du dispositif.

Dans un exemple de réalisation, une étape c) de détermination de la position d'au moins une zone du visage du sujet peut être réalisée par triangulation à l'aide d'au moins deux des première, deuxième et troisième caméras. En particulier, l'étape c) peut être réalisée par triangulation à l'aide de la troisième caméra et de l'une au moins des première et deuxième caméras. L'étape c) peut être réalisée après l'étape b). En variante, l'étape c) est réalisée avant l'étape b). Avant l'étape c), une étape de détection d'au moins une zone du visage, par exemple des yeux, du sujet est réalisée.

La zone du visage dont la position est déterminée durant l'étape c) peut être la zone de la bouche, du nez ou encore être le visage tout entier. Dans un exemple de réalisation, la position des yeux du sujet peut être déterminée par triangulation durant l'étape c).

Dans un exemple de réalisation, une étape d) de reconnaissance d'au moins un iris des yeux peut être effectuée après l'étape c), l'étape d) comportant les étapes suivantes :

d1) acquisition d'au moins une image d'un ou des iris des yeux du sujet par le système de prise de vue d'iris, le système de prise de vue d'iris étant piloté au moins par des données associées à la position des yeux du sujet déterminée lors de l'étape c), et

d2) reconnaissance d'au moins un iris des yeux du sujet par comparaison de données associées à ladite au moins une image d'un ou des iris obtenue lors de l'étape d1) à des données d'iris de référence.

Le pilotage du système de prise de vue d'iris peut-être réalisé par commande électro-mécanique. Le système de prise de vue d'iris peut encore être piloté de manière à sélectionner une image nette dans un flux vidéo obtenu à partir d'une estimation de la distance au sujet. On peut encore utiliser une combinaison de ces deux aspects : pilotage par commande électro-mécanique pour le cadrage, et sélection d'une image pour la netteté.

Cette reconnaissance de l'iris peut être effectuée par le dispositif ou, en variante, par un système informatique externe au dispositif.

En variante ou en combinaison, une étape e) d'estimation d'un modèle 3D du visage du sujet peut être effectuée, après l'étape c), à partir de la position d'au moins une zone du visage obtenue lors de l'étape c).

Dans un exemple de réalisation, l'estimation d'un modèle 3D du visage effectuée lors de l'étape e) peut permettre de réaliser une reconnaissance faciale 3D du sujet. Il est donc possible de mettre en oeuvre un procédé dans lequel on réalise à la fois une reconnaissance faciale 2D du sujet ainsi qu'une reconnaissance faciale 3D du même sujet. Cette reconnaissance faciale 3D peut être effectuée par le dispositif ou, en variante, par un système informatique externe au dispositif.

Une manière d'effectuer la reconnaissance faciale 3D à partir de la position 3D d'une zone du visage est par exemple détaillée au deuxième paragraphe de la page 641 de la publication Kakadiaris, I. A.; Passalis, G. and Toderici, G. and Murtuza, N. and Karampatziakis, N. and Theoharis, T. (2007) : "3D face recognition in the presence of facial expressions: an annotated deformable model approach", IEEE Transactions on Pattern Analysis and Machine Intelligence (PAMI) 13 (12).

L'estimation effectuée lors de l'étape e) peut encore servir à déterminer les images utiles pour la reconnaissance faciale 2D. Ainsi, on peut par exemple après l'étape e) opérer une sélection sur les images prises par les caméras du dispositif en vue de la reconnaissance faciale 2D, cette sélection étant réalisée à partir de données associées à l'estimation réalisée lors de l'étape e), la reconnaissance faciale 2D étant ensuite effectuée à partir des images sélectionnées. L'estimation effectuée lors de l'étape e) peut encore permettre de commander les caméras du dispositif afin d'acquérir les images utiles pour la reconnaissance faciale 2D.

Brève description des dessins

D'autres caractéristiques et avantages de l'invention ressortiront de la description suivante, en référence aux dessins annexés, sur lesquels :

- la figure 1 représente une coupe longitudinale partielle d'un exemple de dispositif selon l'invention,
- la figure 2 représente un dispositif selon l'invention ainsi que le volume d'acquisition en vue de côté,
- la figure 3 représente, de manière très schématique et partielle, un exemple préférentiel d'inclinaison de l'axe optique de la troisième caméra,
- la figure 4 représente une coupe longitudinale partielle d'une variante de dispositif selon l'invention,
- la figure 5 représente une coupe longitudinale partielle d'une variante de dispositif selon l'invention, et
- les figures 6A à 7B comparent les performances d'un dispositif selon l'invention et d'un dispositif hors invention.

Description détaillée de modes de réalisation

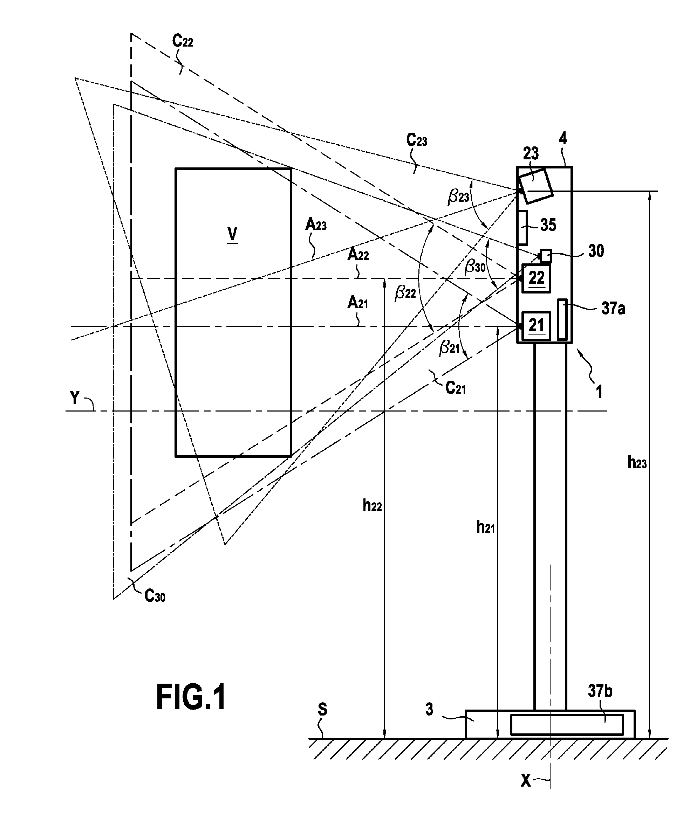

On a représenté à la figure 1 un dispositif 1 selon l'invention. Dans l'exemple illustré, le dispositif 1 se présente sous la forme d'un dispositif fixe présentant dans sa partie inférieure un pied 3 assurant sa stabilité sur la surface support S sur laquelle il est présent. Comme illustré, le dispositif 1 peut avoir une forme de tour. Le dispositif 1 selon l'invention comporte une première, deuxième et troisième caméras 21, 22 et 23. Les caméras sont logées dans un châssis 4 afin d'assurer leur protection. Le dispositif 1 peut avantageusement équiper une porte automatique, par exemple dans un aéroport. On ne sort pas du cadre de l'invention si le dispositif a une forme différente. Dans une variante non illustrée, le dispositif ne comporte pas de pied et est fixé à une paroi à une hauteur convenable pour réaliser l'identification ou l'authentification.

Comme illustré à la figure 1, les première, deuxième et troisième caméras sont décalées en hauteur suivant un axe X de placement des caméras correspondant ici à l'axe d'élongation du dispositif 1. Dans l'exemple illustré, l'axe X de placement des caméras est vertical lorsque le dispositif 1 est dans une configuration de fonctionnement. Plus précisément, la première caméra 21 est placée du côté du pied 3 du dispositif 1 et à une première hauteur $h_{21}$, la deuxième caméra 22 est placée à une deuxième hauteur $h_{22}$ supérieure à la première hauteur $h_{21}$ et la troisième caméra 23 est placée à une troisième hauteur $h_{23}$ supérieure à la deuxième hauteur $h_{22}$. Dans un exemple de réalisation, les caméras 21, 22 et 23 peuvent être alignées verticalement. En variante, les caméras 21, 22 et 23 sont décalées en hauteur suivant l'axe de placement des caméras X et décalées en largeur.

La présence de la deuxième caméra 22 entre les première et troisième caméras 21 et 23 peut avantageusement permettre d'améliorer les performances, pour les personnes les plus grandes pour lesquelles les algorithmes de détection de zones du visage ne donneraient pas d'aussi bons résultats en utilisant la première caméra. En outre, le fait d'utiliser cette deuxième caméra permet en augmentant le nombre de caméras mises en oeuvre de limiter les effets de la projection perspective pour la caméra dont la hauteur est la plus adaptée à la taille du sujet comme c'est le cas dans le système SmartGate® par exemple.

Sauf mention contraire, la hauteur d'une caméra du dispositif 1 correspond à la hauteur à laquelle est placé l'objectif de ladite caméra. On peut, par exemple, avoir les première, deuxième et troisième hauteurs suivantes pour le dispositif selon l'invention : $h_{21}$ = 140 cm, $h_{22}$ = 160 cm et $h_{23}$ = 190 cm.

Plus généralement, le dispositif 1 peut être tel que les trois conditions suivantes soient vérifiées lorsqu'il est dans une configuration de fonctionnement :

- $h_{21}$ est comprise entre 130 cm et 150 cm, $h_{21}$ étant par exemple égale à 140 cm,
- $h_{22}$ est comprise entre 150 cm et 170 cm, $h_{22}$ étant par exemple égale à 160 cm,
- $h_{23}$ est comprise entre 180 cm et 200 cm, $h_{23}$ étant par exemple égale à 190 cm.

De telles valeurs pour $h_{21}$ peuvent être avantageuses notamment lorsque le dispositif 1 selon l'invention équipe une porte d'aéroport afin d'éviter les masquages liés aux éléments existants dans l'aéroport, des positions plus basses risquant d'engendrer des masquages. De telles valeurs pour $h_{21}$ permettent en outre d'obtenir un écartement suffisant avec la troisième caméra pour obtenir une précision de triangulation optimale lorsque la troisième caméra est utilisée pour la triangulation et sont également utiles pour optimiser les performances des algorithmes d'analyse 2D pour les personnes les plus petites dont le regard ne serait pas orienté vers le haut.

De telles valeurs pour $h_{23}$ correspondent avantageusement à un placement de la troisième caméra 23 à une hauteur relativement basse permettant de limiter l'encombrement du dispositif tout en permettant d'obtenir de bonnes performances en termes de triangulation lorsque la troisième caméra 23 est utilisée pour ce faire.

Comme illustré à la figure 1, l'axe optique $A_{21}$ de la première caméra 21 peut être parallèle à l'axe optique $A_{22}$ de la deuxième caméra 22 et l'axe optique $A_{23}$ de la troisième caméra 23 n'est pas parallèle aux axes optiques $A_{21}$ et $A_{22}$ et est dirigé vers ces derniers. Ainsi, l'axe optique $A_{23}$ intersecte les axes optiques $A_{21}$ et $A_{22}$. Dans l'exemple illustré, les axes optiques $A_{22}$ et $A_{23}$ se croisent dans le volume d'acquisition V et les axes optiques $A_{21}$ et $A_{23}$ se croisent à l'extérieur du volume d'acquisition V du côté opposé au dispositif 1. Ainsi, dans l'exemple illustré, les axes optiques $A_{21}$, $A_{22}$ et $A_{23}$ ne sont pas concourants (i.e. ils ne se coupent pas tous les trois au même point).

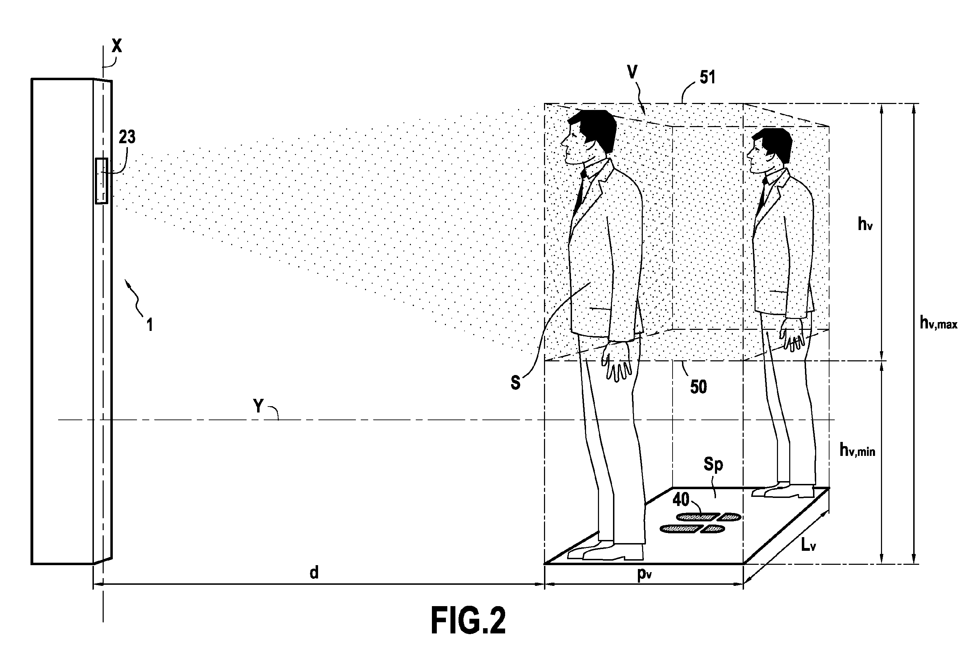

Dans l'exemple illustré à la figure 1, les axes optiques $A_{21}$ et $A_{22}$ sont parallèles à l'axe normal Y perpendiculaire à l'axe de placement des caméras X. L'axe normal Y est dans l'exemple illustré horizontal. L'axe optique $A_{23}$ fait dans l'exemple illustré un angle d'environ 15° avec l'axe normal Y. Plus généralement, l'axe optique $A_{23}$ de la troisième caméra peut faire un angle inférieur à 20° avec l'axe normal Y. Comme illustré à la figure 2, l'axe normal Y est parallèle à la surface de positionnement $S_p$ du sujet S dans le volume d'acquisition V et s'étend entre le dispositif 1 et le volume d'acquisition V. Il est en variante possible que les axes optiques des première et deuxième caméras forment un angle non nul avec l'axe normal, par exemple inférieur ou égal à 10°, voire inférieur ou égal à 5°.

Dans l'exemple illustré à la figure 1, les axes optiques $A_{21}$, $A_{22}$ sont horizontaux et l'axe optique $A_{23}$ s'étend en se rapprochant de la surface de positionnement $S_p$. Une telle configuration permet avantageusement de limiter au maximum l'éblouissement des caméras 21, 22 et 23 par des sources de lumière artificielles situées en hauteur ou par le soleil. Aussi, une telle configuration permet d'améliorer les performances d'identification et d'authentification obtenues pour les sujets de petite taille du fait d'une limitation de l'angle fait entre l'axe optique $A_{21}$ et la normale au visage de tels sujets.

Les champs de chacune des caméras 21, 22, 23 respectivement notés $C_{21}$, $C_{22}$ et $C_{23}$ sont représentés à la figure 1. Ces champs $C_{21}$, $C_{22}$ et $C_{23}$ sont vus en coupe à la figure 1. Dans l'exemple illustré à la figure 1, les angles de champ selon la hauteur des caméras 21, 22 et 23, respectivement notés $\beta_{21}$, $\beta_{22}$ et $\beta_{23}$, sont sensiblement égaux, par exemple égaux à environ 60°. On ne sort pas du cadre de l'invention lorsqu'au moins deux des angles de champ selon la hauteur des caméras sont différents. L'intégralité du volume d'acquisition V est couvert par la superposition des champs des première et deuxième caméras $C_{21}$ et $C_{22}$. En d'autres termes, le volume d'acquisition est couvert sur toute sa hauteur, toute sa largeur et toute sa profondeur par la superposition des champs des première et deuxième caméras. L' « addition » des champs $C_{21}$ et $C_{22}$ permet de couvrir l'intégralité du volume d'acquisition.

L'inclinaison de l'axe optique $A_{23}$ peut avantageusement être choisie de manière à obtenir une couverture du volume d'acquisition V maximale par le champ $C_{23}$. Une telle configuration permet avantageusement de réaliser, à l'aide de la troisième caméra 23 et de l'une au moins des première et deuxième caméras 21 et 22, une triangulation dans une zone du volume d'acquisition V de volume maximal. En particulier, l'intégralité du volume d'acquisition V peut être couverte par le champ $C_{23}$ de la troisième caméra 23. Dans ce cas, l'angle de champ de la troisième caméra selon la largeur est suffisant pour couvrir toute la largeur $L_v$ du volume d'acquisition V. En outre, il est avantageux de se placer dans une configuration dans laquelle l'inclinaison de l'axe optique $A_{23}$ est minimale tout en permettant de couvrir l'intégralité du volume d'acquisition V par le champ $C_{23}$ de la troisième caméra 23. Une telle configuration permet (i) de réaliser une triangulation dans tout le volume d'acquisition V, et (ii) d'obtenir de très bonnes performances pour la reconnaissance faciale de sujets de grande taille, l'axe optique $A_{23}$ formant dans ce cas un angle limité avec la normale au visage des sujets.

Le dispositif 1 comporte, en outre, un support de stockage d'informations dans lequel sont stockées des informations associées à au moins une caractéristique biométrique d'au moins un sujet ou apte à stocker de telles informations. Le dispositif 1 est équipé de moyens de traitement permettant de réaliser, à partir du flux vidéo provenant des caméras 21, 22 et 23, la reconnaissance faciale du sujet et de déterminer la position d'au moins une zone du visage du sujet par triangulation. Le dispositif 1 comporte ainsi un système de traitement informatique 37a et 37b comportant notamment un ordinateur configuré pour traiter les données obtenues lors du fonctionnement du dispositif 1.

Dans une variante non illustrée, le support de stockage d'informations peut être présent dans un système informatique externe au dispositif, le dispositif étant alors configuré pour échanger des données avec ledit système informatique. De la même manière, la comparaison biométrique en vue par exemple d'effectuer une reconnaissance faciale du sujet peut être réalisée par un système informatique externe au dispositif. Dans ce cas, le dispositif réalise la capture d'images et transmet des données au système informatique externe afin qu'il effectue l'identification ou l'authentification.

Diverses méthodes de reconnaissance faciale peuvent être mises en oeuvre dans le cadre de l'invention. A ce sujet, on peut se référer à la publication Zhao et al. « Face recognition : A Literature Survey » ; ACM Computing Surveys, Vol. 35, No. 4, Décembre 2003, pp. 399-458 qui cite diverses méthodes de reconnaissance faciale.

Le dispositif 1 illustré à la figure 1 comporte, en outre, un système de prise de vue d'iris 30 configuré pour prendre au moins une image d'un ou des iris des yeux du sujet une fois la triangulation de la position des yeux du sujet effectuée. Le dispositif 1 peut ainsi comporter, en plus des caméras 21, 22 et 23, un système de prise de vue d'iris 30 comportant une caméra d'iris. Le champ $C_{30}$ du système de prise de vue d'iris 30 recouvre avantageusement l'intégralité du volume d'acquisition V comme illustré à la figure 1. Le système de prise de vue d'iris 30 peut, comme illustré, être situé au-dessus des première et deuxième caméras 21 et 22. L'angle de champ selon la hauteur $\beta_{30}$ du système de prise de vue d'iris 30 peut être différent de, ou en variante être égal à, l'un au moins des angles de champ $\beta_{21}$, $\beta_{22}$ et $\beta_{23}$. Dans l'exemple illustré à la figure 1, l'angle de champ $\beta_{30}$ est d'environ 60°.

Le système de prise de vue d'iris 30 est configuré pour être piloté au moins par des données associées à la position des yeux du sujet déterminée dans l'exemple illustré à la figure 1 par triangulation à l'aide de la troisième caméra 23 et de l'une au moins des première et deuxième caméras 21 et 22. Le système de prise de vue d'iris 30 peut être électro-mécanique. Comme mentionné plus haut, le système de prise de vue d'iris 30 peut être en communication avec un système de reconnaissance d'iris configuré pour réaliser la reconnaissance d'au moins un iris des yeux du sujet, ce système de reconnaissance d'iris étant intégré au dispositif 1 ou situé à l'extérieur de ce dernier.

L'exemple illustré à la figure 1 montre le cas d'un système de prise de vue d'iris 30 intégré au dispositif 1 mais on ne sort pas du cadre de la présente invention si le système de prise de vue d'iris est extérieur au dispositif. Le dispositif 1 illustré à la figure 1 comporte, en outre, un écran 35 d'interface homme-machine destiné à attirer le regard du sujet afin d'optimiser les performances de reconnaissance faciale et iris du dispositif 1. L'écran 35 peut, en outre, permettre d'afficher des informations relatives par exemple à l'avancement ou au résultat du procédé d'identification ou d'authentification. L'écran 35 peut comme illustré à la figure 1 être situé au-dessus de la deuxième caméra 22 et en dessous de la troisième caméra 23 lorsque le dispositif 1 est dans une configuration de fonctionnement.

L'exemple illustré met en oeuvre trois caméras pour effectuer la reconnaissance faciale et la triangulation mais on ne sort pas du cadre de l'invention si l'on utilise un nombre supérieur de caméras. Il est, par exemple, possible qu'un dispositif comporte une troisième et une quatrième caméras ayant chacune un axe optique formant un angle non nul avec les axes optiques des première et deuxième caméras et intersectant ces derniers.

On a représenté à la figure 2 en vue de côté un dispositif 1 selon l'invention ainsi que le volume d'acquisition V dans lequel un sujet S est positionné pour procéder à son identification ou à son authentification. Nous allons donner dans la suite, à titre d'exemple, des valeurs possibles pour les dimensions du volume d'acquisition V. Sauf mention contraire, les hauteurs relatives au volume d'acquisition $h_{v,min}$, $h_{v,max}$ et $h_v$ qui vont être décrites sont mesurées perpendiculairement à la surface de positionnement $S_p$.

La hauteur $h_{v,min}$ de la frontière inférieure 50 du volume d'acquisition V peut être supérieure ou égale à 80 cm, par exemple être comprise entre 80 cm et 120 cm. A titre d'exemple, la hauteur $h_{v,min}$ de la frontière inférieure 50 du volume d'acquisition est égale à 100 cm.

La hauteur $h_{v,max}$ de la frontière supérieure 51 du volume d'acquisition V peut être supérieure ou égale à 190 cm, par exemple être comprise entre 190 cm et 210 cm. A titre d'exemple, la hauteur $h_{v,max}$ de la frontière supérieure 51 du volume d'acquisition V est égale à 200 cm.

La hauteur $h_v$ du volume d'acquisition V, correspondant à $h_{v,max}$ - $h_{v,min}$, peut être supérieure ou égale à 70 cm, par exemple être comprise entre 70 cm et 130 cm. A titre d'exemple, la hauteur $h_v$ du volume d'acquisition V est égale à 100 cm.

La profondeur $p_v$ du volume d'acquisition V, mesurée selon l'axe normal Y, peut être supérieure ou égale à 20 cm, par exemple être comprise entre 20 cm et 80 cm. A titre d'exemple, la profondeur $p_v$ du volume d'acquisition V peut être égale à 40 cm.

La largeur $L_v$ du volume d'acquisition V, mesurée perpendiculairement à l'axe normal Y et à l'axe de placement des caméras X, peut être supérieure ou égale à 30 cm, par exemple être comprise entre 30 cm et 80 cm. A titre d'exemple, la largeur $L_v$ peut être égale à 50 cm.

La distance d, mesurée selon l'axe normal Y, entre la troisième caméra 23 et le volume d'acquisition V peut être supérieure ou égale à 30 cm, par exemple être comprise entre 30 cm et 250 cm.

Nous allons à présent décrire des exemples de procédés d'identification ou d'authentification d'un sujet selon l'invention.

Dans une première étape a), le sujet S se positionne dans le volume d'acquisition V. Pour aider le sujet S à se positionner, la position du volume d'acquisition V peut être repérée par un indicateur visible 40, par exemple formé, comme dans l'exemple illustré à la figure 2, sur la surface de positionnement $S_p$ sur laquelle le sujet S est destiné à se placer. On peut utiliser d'autres moyens pour repérer le volume d'acquisition comme une délimitation physique de tout ou partie du volume d'acquisition par un ou plusieurs éléments.

Après positionnement du sujet S dans le volume d'acquisition V, un traitement de reconnaissance faciale 2D du sujet S est effectué à l'aide d'un dispositif 1 tel que décrit plus haut (étape b)). La reconnaissance faciale 2D peut être réalisée à partir des images fournies par l'une au moins des première et deuxième caméras 21 et 22. En variante ou en combinaison, la reconnaissance faciale 2D peut être réalisée au moins à partir des images fournies par la troisième caméra 23. Cette dernière possibilité peut être d'intérêt pour identifier ou authentifier des sujets relativement grands.

Afin de réaliser la reconnaissance faciale 2D, des données associées aux images fournies par la ou les caméras mise(s) en oeuvre peuvent être comparées à des données de référence stockées sur un support de stockage d'information.

Après positionnement du sujet S dans le volume d'acquisition V, une étape c) de détermination par triangulation de la position d'au moins une zone du visage, par exemple des yeux, du sujet est effectuée à partir des images fournies par deux au moins des première, deuxième et troisième caméras. L'étape c) est initiée par une sous-étape consistant à détecter la zone du visage dans au moins un flux vidéo 2D fourni par l'une au moins des caméras. Ainsi, le fait de minimiser les angles entre les axes optiques des caméras et la normale au visage du sujet pour la reconnaissance faciale 2D est également avantageux pour la réalisation de l'étape de triangulation. Comme mentionné plus haut, l'étape c) peut être réalisée avant ou après l'étape b).

Une fois la position de la zone du visage du sujet S déterminée par triangulation, on peut réaliser un pilotage, par exemple par le biais d'un système électromécanique, d'un système de prise de vue d'iris afin de réaliser la reconnaissance d'au moins un iris des yeux du sujet à l'aide d'un système de reconnaissance d'iris en communication avec ledit système de prise de vue d'iris.

On peut encore réaliser, à partir de la position d'au moins une zone du visage obtenue, l'estimation d'un modèle 3D du visage du sujet afin de réaliser une reconnaissance faciale 3D du sujet.

Les reconnaissances iris ou faciale 3D peuvent être réalisées par le dispositif lui-même ou, en variante, par un système informatique externe au dispositif.

L'utilisation d'un même ensemble de caméras afin de réaliser la reconnaissance faciale 2D et la stéréoscopie 3D permet de mutualiser les traitements de détection et de réduire les besoins en termes de calcul conduisant ainsi avantageusement à une diminution du coût de mise en oeuvre du procédé ainsi qu'à une diminution de l'encombrement notamment du fait de la possibilité de réduire le nombre de caméras nécessaires pour des performances similaires.

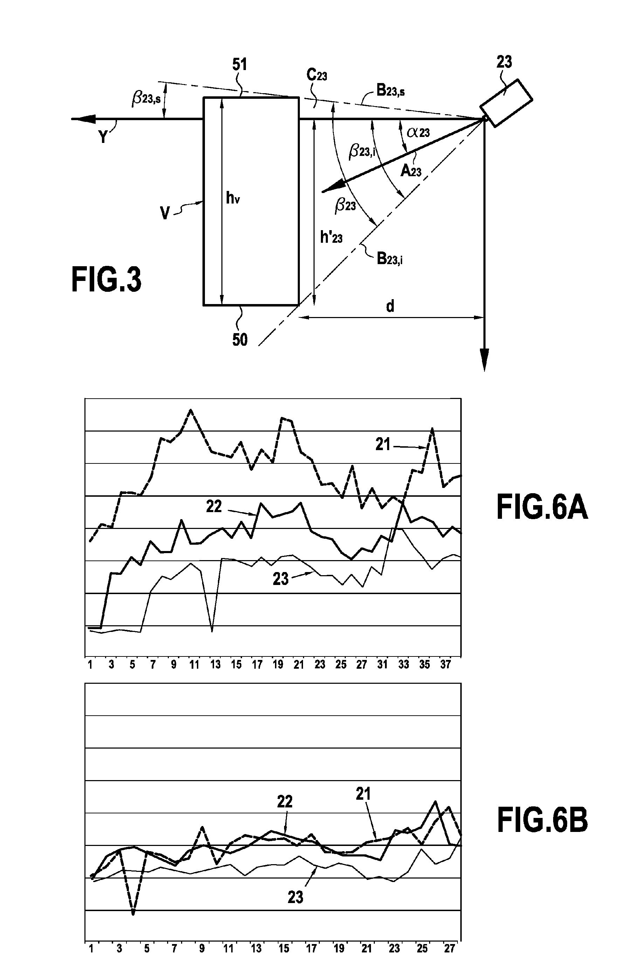

Nous allons à présent détailler la figure 3 et déterminer la valeur optimale pour l'angle $\alpha_{23}$ que fait l'axe optique $A_{23}$ de la troisième caméra 23 avec l'axe normal Y.

A la figure 3, les notations sont les mêmes que précédemment. Il a, en outre, été introduit :

- $h'_{23}$ qui désigne la hauteur de la troisième caméra 23 mesurée par rapport à la hauteur de la frontière inférieure 50 du volume d'acquisition V,
- l'angle $\beta_{23,i}$ qui correspond à l'angle formé par le bord $B_{23,i}$ de champ inférieur de la troisième caméra 23 avec l'axe normal Y,
- l'angle $\beta_{23,s}$ qui correspond à l'angle formé par le bord $B_{23,s}$ de champ supérieur de la troisième caméra 23 avec l'axe normal Y.

Nous détaillons ci-dessous le calcul permettant d'obtenir l'expression analytique de la valeur minimale de l'angle de l'axe optique de la troisième caméra permettant de couvrir l'intégralité du volume d'acquisition V afin de permettre la triangulation 3D dans tout le volume à l'aide de cette troisième caméra. Comme expliqué plus haut, le fait d'avoir une inclinaison minimale pour l'axe optique de la troisième caméra permet avantageusement d'optimiser les traitements de reconnaissance faciale réalisés à partir des images fournies par cette caméra.

Nous avons :

$$\beta_{23,i} = \text{arctg}(h'_{23}/d)$$

$$\alpha_{23} = \beta_{23,i} - \beta_{23}/\,2$$

Ce qui permet d'en déduire le résultat recherché :

$$\alpha_{23} = \text{arctg}(h'_{23}/d) - \beta_{23}/\,2$$

Une condition supplémentaire s'ajoute sur l'angle de champ $\beta_{23}$ puisqu'il est nécessaire afin de pouvoir effectuer la triangulation dans tout le volume d'acquisition V à l'aide de la troisième caméra que l'angle de champ $\beta_{23}$ soit suffisamment grand pour que le champ $C_{23}$ de la caméra 23 puisse couvrir tout le volume d'acquisition V. D'où la condition :

$$\beta_{23} \geq \text{arctg}(h'_{23}/d) + \text{arctg}((h_v\text{-}h'_{23})/d)$$

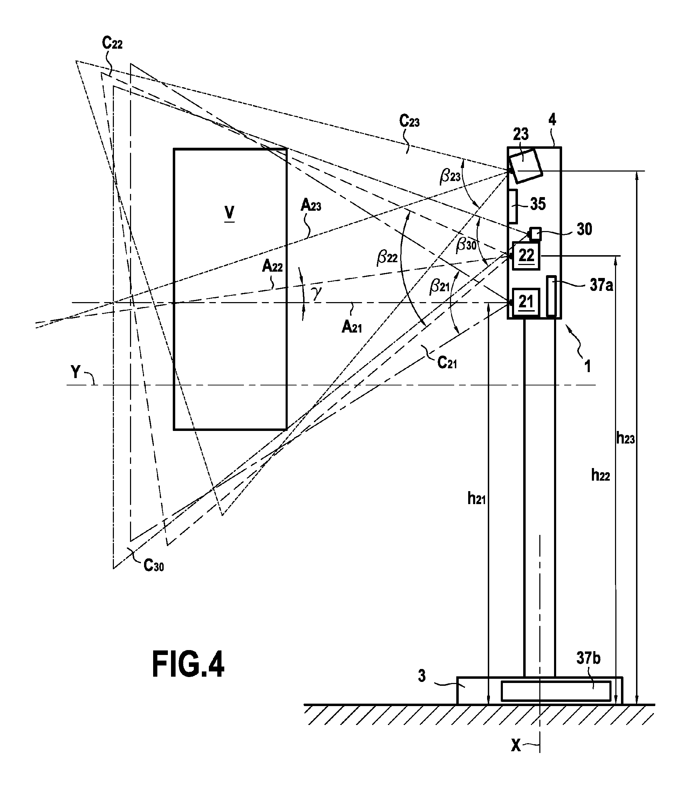

On a représenté à la figure 4 un exemple de variante de réalisation de dispositif selon l'invention. La seule différence entre les dispositifs illustrés aux figures 1 et 4 est que, à la figure 4, l'axe optique $A_{22}$ de la deuxième caméra 22 est légèrement incliné vers le bas (i.e. l'axe optique $A_{22}$ de la deuxième caméra 22 s'étend en se rapprochant de la surface de positionnement sur laquelle le sujet est destiné à être présent). L'axe optique $A_{22}$ forme un angle $\gamma$ non nul avec l'axe optique $A_{21}$ de la première caméra 21. L'angle $\gamma$ est inférieur à 10°, par exemple inférieur ou égal à 5°. Le dispositif 1 illustré à la figure 4 permet de déterminer par triangulation la position d'au moins une zone du visage, par exemple des yeux, du sujet à l'aide des première 21 et deuxième 22 caméras. On peut encore utiliser la troisième caméra 23 et l'une au moins des première 21 et deuxième 22 caméras pour réaliser cette triangulation dans le dispositif 1 illustré à la figure 4.

Bien entendu, on ne sort pas du cadre de l'invention lorsque l'axe optique de la deuxième caméra est parallèle à l'axe normal et l'axe optique de la première caméra s'étend en se rapprochant de la surface de positionnement sur laquelle le sujet est destiné à être présent. Dans une variante non illustrée, les axes optiques des première et deuxième caméras s'étendent chacun en se rapprochant de la surface de positionnement sur laquelle le sujet est destiné à être présent.

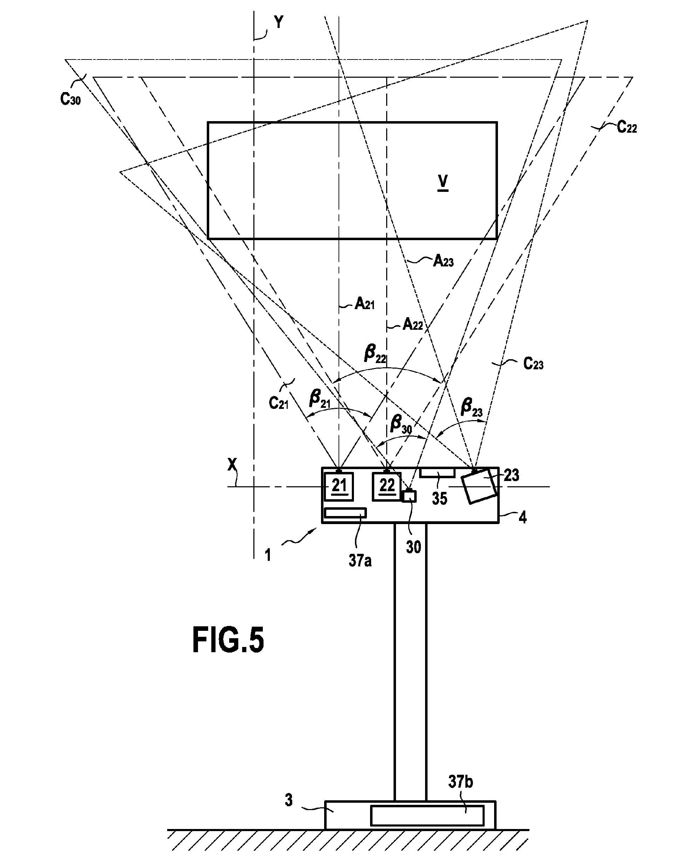

On a représenté à la figure 5 une variante de dispositif selon l'invention. L'axe de placement X des caméras est, dans l'exemple illustré, parallèle à la surface support S sur laquelle le dispositif 1 est placé, le dispositif 1 étant dans ce cas « tourné vers le haut ». Les première 21, deuxième 22 et troisième 23 caméras sont successivement décalées le long de l'axe X. A la différence du cas illustré à la figure 1, l'axe de placement X est cette fois horizontal et l'axe normal Y vertical. Lors de l'utilisation de ce dispositif 1 illustré à la figure 5, un utilisateur se penche au-dessus de ce dispositif 1 de manière à placer son visage dans le volume d'acquisition V afin de procéder à l'identification ou à l'authentification.

On a représenté aux figures 6A et 6B un résultat d'essai comparatif entre un dispositif selon l'invention (figure 6A) et un dispositif hors invention (figure 6B). La variable en abscisse des graphes des figures 6A et 6B correspond à l'index de l'image dans une séquence vidéo réalisée avec une personne de petite taille. L'ordonnée similaire entre les deux graphes correspond à un score de comparaison biométrique. La seule différence entre le dispositif hors invention et le dispositif selon l'invention évalués dans cet essai comparatif est relative à l'angle que fait l'axe optique de la première caméra avec l'axe normal. En effet, dans l'exemple de dispositif selon l'invention évalué, les axes optiques des première et deuxième caméras sont tous les deux parallèles à l'axe normal et l'axe optique de la troisième caméra intersecte ces derniers. En revanche, dans le dispositif hors invention évalué l'axe optique de la première caméra est incliné vers le haut et fait un angle de 10° avec l'axe normal et l'axe optique de la deuxième caméra est parallèle à l'axe normal (dans le dispositif hors invention, les axes optiques des première et deuxième caméras ne forment donc pas entre eux un angle strictement inférieur à 10°). Ainsi, dans le dispositif selon l'invention évalué, aucun des axes optiques des caméras ne s'étend vers le haut et les axes optiques des première et deuxième caméras sont horizontaux.

La première caméra d'un tel dispositif selon l'invention réalise une bien meilleure reconnaissance des sujets de petite taille par rapport au dispositif hors invention (voir la courbe relative à la première caméra : caméra du bas, courbe 21). Comme déjà mentionné plus haut, un tel résultat s'explique par le fait que, dans la configuration selon l'invention évaluée, l'angle formé entre la normale au visage des sujets de petite taille et l'axe optique de la première caméra est minimal et par le fait que l'axe optique de la première caméra n'est pas orienté vers le haut afin de limiter au maximum l'éblouissement de celle-ci par exemple par des éclairages situés en hauteur.

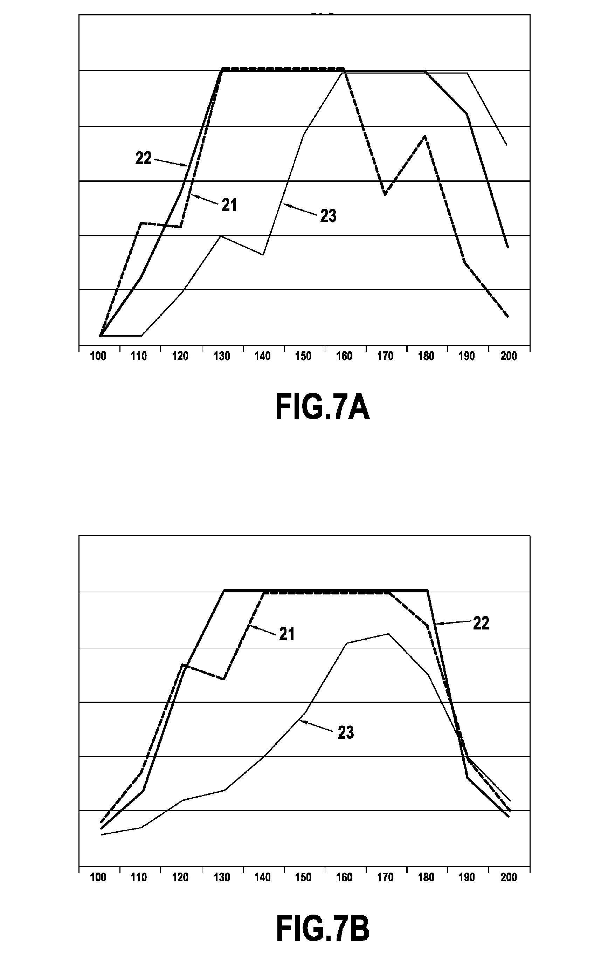

Les figures 7A et 7B représentent aussi des résultats d'essai comparatif entre un dispositif selon l'invention et un dispositif hors invention. Les abscisses de ces figures représentent la taille en cm des sujets évalués. Dans le dispositif selon l'invention évalué (figure 7A), les axes optiques des première et deuxième caméras sont horizontaux alors que dans le dispositif hors invention évalué (figure 7B) l'axe optique de la première caméra est incliné vers le haut et fait un angle de 10° avec l'axe normal et l'axe optique de la deuxième caméra est parallèle à l'axe normal. En plus de cette différence, il y a une différence dans l'inclinaison de l'axe optique de la troisième caméra dans les dispositifs évalués. L'inclinaison de l'axe optique de la troisième caméra dans le dispositif hors invention évalué est supérieure à l'inclinaison de l'axe optique de la troisième caméra dans le dispositif selon l'invention évalué de telle manière à faire en sorte que les axes optiques des trois caméras soient concourants à l'extérieur du volume d'acquisition dans le dispositif hors invention évalué.

Dans le dispositif selon l'invention évalué, l'inclinaison de l'axe optique de la troisième caméra correspond à la valeur minimale permettant la réalisation d'une triangulation 3D à l'aide de la troisième caméra dans l'intégralité du volume d'acquisition V ($\alpha_{23}$ = arctg(h'$_{23}$/d) - $\alpha_{23}$/ 2 comme décrit en lien avec la figure 3).

La comparaison des figures 7A et 7B montre notamment que le dispositif selon l'invention présente de meilleures performances pour la reconnaissance de sujets de grande taille du fait d'un angle réduit entre la normale au visage de tels sujets et l'axe optique de la troisième caméra (voir résultats obtenus avec la caméra du haut, courbe 23). On observe aussi que le dispositif selon l'invention présente de meilleures performances pour la reconnaissance de sujets de petite taille à l'aide de la caméra du bas 21 (voir résultats obtenus avec la caméra du bas, courbe 21).

L'expression « comportant/contenant/comprenant un(e) » doit se comprendre comme « comportant/contenant/comprenant au moins un(e) ».

L'expression « compris(e) entre ... et ... » ou « allant de ... à ... » doit se comprendre comme incluant les bornes.

**Revendications**

1. Dispositif (1) destiné à être utilisé pour identifier ou authentifier un sujet (S) positionné dans un volume d'acquisition (V) à partir d'au moins une caractéristique biométrique du sujet (S), le dispositif (1) comportant successivement, de manière décalée le long d'un axe (X) de placement de caméras, une première caméra (21), une deuxième caméra (22) et une troisième caméra (23), les axes optiques (A$_{21}$ ; A$_{22}$) des première et deuxième caméras formant entre eux un angle strictement inférieur à 10° et l'axe optique (A$_{23}$) de la troisième caméra intersectant les axes optiques (A$_{21}$ ; A$_{22}$) des première et deuxième caméras, les axes optiques (A$_{21}$ ; A$_{22}$) des première et deuxième caméras formant chacun un angle inférieur ou égal à 5° avec un axe normal (Y) perpendiculaire à l'axe (X) de placement des caméras.

2. Dispositif (1) selon la revendication 1, **caractérisé en ce que** les axes optiques (A$_{21}$ ; A$_{22}$) des première et deuxième caméras sont sensiblement parallèles entre eux.

3. Dispositif (1) selon la revendication 2, **caractérisé en ce que** les axes optiques (A$_{21}$ ; A$_{22}$) des première et deuxième caméras sont chacun parallèles à l'axe normal (Y).

4. Dispositif (1) selon l'une quelconque des revendications 1 à 3, **caractérisé en ce qu'**il comporte, en plus des première (21), deuxième (22) et troisième caméras (23), un système (30) de prise de vue d'iris intégré en communication avec un système de reconnaissance d'iris, ledit système de reconnaissance d'iris étant configuré pour réaliser la reconnaissance d'au moins un iris des yeux du sujet (S) à partir d'au moins une image d'un ou des iris des yeux du sujet (S) prise par le système (30) de prise de vue d'iris.

**5.** Dispositif (1) selon la revendication 4, **caractérisé en ce que** le système (30) de prise de vue est un système de prise de vue d'une partie de la texture d'au moins un iris.

**6.** Dispositif (1) selon l'une quelconque des revendications 1 à 5, **caractérisé en ce que** :

- la première caméra (21) est placée à une première hauteur $h_{21}$,
- la deuxième caméra (22) est placée à une deuxième hauteur $h_{22}$ supérieure à la première hauteur $h_{21}$, et
- la troisième caméra (23) est placée à une troisième hauteur $h_{23}$ supérieure à la deuxième hauteur $h_{22}$.

**7.** Dispositif (1) selon la revendication 6, **caractérisé en ce que** l'axe optique $(A_{23})$ de la troisième caméra (23) s'étend en se rapprochant d'une surface de positionnement $(S_p)$ sur laquelle le sujet (S) est destiné à être présent pour procéder à l'identification ou à l'authentification et **en ce que** les axes optiques $(A_{21} ; A_{22})$ des première et deuxième caméras sont chacun parallèles à l'axe normal (Y).

**8.** Dispositif (1) selon la revendication 6, **caractérisé en ce que** l'axe optique $(A_{23})$ de la troisième caméra (23) et l'un au moins des axes optiques $(A_{21} ; A_{22})$ des première et deuxième caméras s'étendent en se rapprochant d'une surface de positionnement $(S_p)$ sur laquelle le sujet (S) est destiné à être présent pour procéder à l'identification ou à l'authentification.

**9.** Dispositif (1) selon l'une quelconque des revendications 6 à 8, **caractérisé en ce que** la superposition des champs $(C_{21} ; C_{22})$ des première et deuxième caméras recouvre l'intégralité du volume d'acquisition (V) et **en ce que** les conditions suivantes sont vérifiées :

- l'angle $\alpha_{23}$ formé entre l'axe optique $(A_{23})$ de la troisième caméra et l'axe normal (Y) perpendiculaire à l'axe de placement (X) des caméras est tel que :

$$(arctg(h'_{23}/d) - \beta_{23}/2) \leq \alpha_{23} \leq 1{,}1*(arctg(h'_{23}/d) - \beta_{23}/2),$$

et
- l'angle $\beta_{23}$ de champ selon la hauteur de la troisième caméra (23) est tel qu'il vérifie la condition suivante :

$$\beta_{23} \geq 2 * (\alpha_{23} + arctg((h_v - h'_{23})/d)),$$

où $h'_{23}$ désigne la hauteur de la troisième caméra (23) mesurée par rapport à la hauteur de la frontière inférieure (50) du volume d'acquisition (V), $h_v$ désigne la hauteur du volume d'acquisition (V), d désigne la distance mesurée selon l'axe normal (Y) entre la troisième caméra (23) et le volume d'acquisition (V).

**10.** Porte automatique équipée d'un dispositif (1) selon l'une quelconque des revendications 1 à 9.

**11.** Procédé d'identification ou d'authentification d'un sujet (S) mettant en oeuvre un dispositif (1) selon l'une quelconque des revendications 1 à 9 ou une porte automatique selon la revendication 10, le procédé comportant les étapes suivantes :

a) positionnement du sujet (S) dans le volume d'acquisition (V), et
b) réalisation d'une reconnaissance faciale 2D du sujet (S) à partir d'une ou plusieurs images du sujet (S) prises par l'une au moins des première (21), deuxième (22) et troisième caméras (23).

**12.** Procédé selon la revendication 11, **caractérisé en ce qu'**une étape c) de détermination de la position d'au moins une zone du visage du sujet (S) est réalisée par triangulation à l'aide d'au moins deux des première, deuxième et troisième caméras.

**13.** Procédé selon la revendication 12, **caractérisé en ce que** la position des yeux du sujet (S) est déterminée par triangulation durant l'étape c).

**14.** Procédé selon la revendication 13, **caractérisé en ce qu'**une étape d) de reconnaissance d'au moins un iris des yeux est effectuée après l'étape c), l'étape d) comportant les étapes suivantes :

d1) acquisition d'au moins une image d'un ou des iris des yeux du sujet (S) par le système de prise de vue d'iris (30), le système de prise de vue d'iris (30) étant piloté au moins par des données associées à la position des yeux du sujet (S) déterminée lors de l'étape c), et
d2) reconnaissance d'au moins un iris des yeux du sujet (S) par comparaison de données associées à ladite au moins une image d'un ou des iris obtenue lors de l'étape d1) à des données d'iris de référence.

**15.** Procédé selon l'une quelconque des revendications 12 à 14, **caractérisé en ce qu'**une étape e) d'estimation d'un modèle 3D du visage du sujet (S) est effectuée, après l'étape c), à partir de la position d'au moins une zone du visage obtenue lors de l'étape c).

FIG.1

**FIG.2**

EP 3 000 397 A1

FIG.3

FIG.6A

FIG.6B

**FIG.4**

**FIG.5**

**FIG.7A**

**FIG.7B**

Europäisches
Patentamt

European
Patent Office

Office européen
des brevets

# RAPPORT DE RECHERCHE EUROPEENNE

Numéro de la demande

EP 15 18 2665

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | SHIHONG LAO ET AL: "3D template matching for pose invariant face recognition using 3D facial model built with isoluminance line based stereo vision", PATTERN RECOGNITION, 2000. PROCEEDINGS. 15TH INTERNATIONAL CONFERENCE ON SEPTEMBER 3-7, 2000; [PROCEEDINGS OF THE INTERNATIONAL CONFERENCE ON PATTERN RECOGNITION. (ICPR)], LOS ALAMITOS, CA, USA,IEEE COMPUT. SOC, US, vol. 2, 3 septembre 2000 (2000-09-03), pages 911-916, XP010533961, ISBN: 978-0-7695-0750-7 | 1-4,6-15 | INV. A61B5/117 G06K9/00 A61B5/00 H04N7/18 G06K9/20 |
| Y | * abrégé * * figures 2, 3 * * page 912 - page 915 * ----- | 5 | |
| X | US 2010/253816 A1 (HANNA KEITH J [US]) 7 octobre 2010 (2010-10-07) | 1-4,6-11 | |
| Y | * figures 1, 3 * | 5 | |
| A | * alinéas [0002], [0005] - [0007] * ----- | 12-15 | DOMAINES TECHNIQUES RECHERCHES (IPC) |
| Y | HAIYUAN WU ET AL: "Glasses frame detection with 3D hough transform", PATTERN RECOGNITION, 2002. PROCEEDINGS. 16TH INTERNATIONAL CONFERENCE ON QUEBEC CITY, QUE., CANADA 11-15 AUG. 2002, LOS ALAMITOS, CA, USA,IEEE COMPUT. SOC, US, vol. 2, 11 août 2002 (2002-08-11), pages 346-349, XP010613891, ISBN: 978-0-7695-1695-0 * figure 1 * * page 347, colonne 1, ligne 14 - ligne 15 * ----- -/-- | 5 | A61B G06K H04N |

Le présent rapport a été établi pour toutes les revendications

| Lieu de la recherche | Date d'achèvement de la recherche | Examinateur |
|---|---|---|
| La Haye | 10 novembre 2015 | Almeida, Mariana |

Europäisches
Patentamt

European
Patent Office

Office européen
des brevets

**RAPPORT DE RECHERCHE EUROPEENNE**

Numéro de la demande

EP 15 18 2665

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | KEN HUGHES ET AL: "Detection of Contact-Lens-Based Iris Biometric Spoofs Using Stereo Imaging", SYSTEM SCIENCES (HICSS), 2013 46TH HAWAII INTERNATIONAL CONFERENCE ON, IEEE, 7 janvier 2013 (2013-01-07), pages 1763-1772, XP032343816, DOI: 10.1109/HICSS.2013.172 ISBN: 978-1-4673-5933-7 * abrégé * * page 1766 - page 1769 * * figures 3-5 * ----- | 5 | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| La Haye | 10 novembre 2015 | Almeida, Mariana |

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 15 18 2665

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-11-2015

| Document brevet cité au rapport de recherche | Date de publication | Membre(s) de la famille de brevet(s) | Date de publication |
|---|---|---|---|
| US 2010253816 A1 | 07-10-2010 | US 2010253816 A1<br>US 2012219279 A1<br>US 2014140685 A1 | 07-10-2010<br>30-08-2012<br>22-05-2014 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **KAKADIARIS, I. A. ; PASSALIS, G. ; TODERICI, G. ; MURTUZA, N. ; KARAMPATZIAKIS, N. ; THE-OHARIS, T.** 3D face recognition in the presence of facial expressions: an annotated deformable model approach. *IEEE Transactions on Pattern Analysis and Machine Intelligence,* 2007, vol. 13 (12

- **ZHAO et al.** Face recognition : A Literature Survey. *ACM Computing Surveys,* Décembre 2003, vol. 35 (4), 399-458